# EUROPEAN PATENT APPLICATION

(11) **EP 0 720 833 A1**
(43) Date of publication of application: **10.07.1996**
(21) Application number: 95850220.5
(22) Date of filing: 07.12.1995
(51) Int. Cl.: A61B 10/00, A61F 2/02, A61L 27/00

(54) **Device for studying and/or influencing the anchorage of implants in living biological tissue**

(30) Priority: 09.12.1994 SE 9404300
(71) Applicant: Nobelpharma AB, S-402 26 Göteborg (SE)
(72) Inventor: Kasemo Bengt, S-464 00 Mellerud (SE); Bokedal Anna, S-411 27 Göteborg (SE); Lausmaa Jukka, S-415 03 Göteborg (SE)
(74) Representative: Olsson, Gunnar

(57) **Abstract**

The invention relates to a device for studying and/or influencing the anchorage of implants in living biological tissue, especially hard tissue, as a response to locally supplied substances. The device consists of a anchoring element (1) for fastening in the tissue and has a central recess (3) in the form of a depot for supplying a substance whose effect on the anchorage of the implant in the tissue is to be studied or utilized. The surface of the anchoring element (1) adjacent to the tissue consists wholly or in part of a permeable material, "filter", which allows added substances to diffuse out directly onto the implant surface, or into the biological fluid around the implant surface, from the central recess (3). The anchoring element resembles clinically employed implants, or implants which are employed within research and development, as regards material and/or surface properties and placing, and also as regards site and surgical technique.

## Description

The invention relates to a device for studying and/or influencing the anchorage of implant materials in living biological tissue, especially hard tissue.

The device consists of an anchoring element whose outer delimitation surface is, after the implantation, in direct contact with the biological host system and whose interior has a recess - a depot - for the supply of a substance whose effect on the anchorage in the tissue is to be studied or utilized.

### BACKGROUND

It is already known to permanently anchor implants directly in bone tissue. In order to avoid loosening, attempts are made to achieve a direct contact, i. e. direct adaption between the implant and the surrounding bone tissue, so-called osseointegration. Such a direct adaptation can be achieved by means of suitable implant design combined with a refined surgical technique. The osseointegration principle which was developed by Professor Brånemark and coworkers has been used clinically for more then 25 years, with good results, for dental constructions which are anchored in the jaw bone.

The principle is based on the implant being made of pure titanium, at least in the boundary zone between living tissue and implant. Swedish Patent 79.02035-0 also describes the importance of the titanium surface structure for obtaining a strong anchorage of the implant in the living bone tissue. Use of an implant having a special, so-called micropitted, surface structure improves still further the prospect of achieving a long-term anchorage of the implant in the body tissue.

In that which follows, anchorage is understood to mean, on the one hand, the course of events by which the implant is anchored, i.e. the incorporation of the implant, and, on the other hand, the long-term stability of the implant on the implantation site.

Currently, there are major deficiencies and gaps in our systematic knowledge of how the incorporation response is influenced by specific properties of the implant surface and by other factors which can be regulated externally. There is, therefore, a great need of methods for studying the incorporation process which include the concomitant possibility of influencing this process with the aid of controllable, external factors including the nature of the surface and also chemical additives.

It is already known to study the growth of bone into implants under standardized conditions SE 461 499. In such studies, a special test chamber is located surgically in the bone tissue, with the chamber being provided with a through channel into which the bone tissue can grow. The test chamber is designed in such a way that it is possible for a test substance in liquid form to be supplied locally to the said channel in the test chamber via a diffusion capillary in order to study how the ingrowth into the cavity can be influenced by the different test substances. After the bone has been growing in for a certain period of time, the test chamber is opened and the newly formed bone tissue in the form of cylindrical rods is removed, measured and evaluated.

However, the above-described bone ingrowth chamber is far from being ideal for studying the incorporation properties of different implant surfaces. It is probable that general incorporation can be influenced by a variety of different factors. While topography is one factor which exerts an effect, SE 79.02035-0, leakage of ions or particles from the implant surface can , for example, also be factors which are of importance for the incorporation. Normally, it is very difficult to manufacture traditional test implants in which one individual factor can be systematically varied without simultaneously producing changes in other parameters. It is then also difficult to draw definite conclusions with regard to any differences which have been observed in biological experiments. Consequently, it is very important, in experimental evaluations, to be able to isolate one individual factor, for example ion release, from other parameters. As an example, it can be mentioned that a so-called bioactive material such as hydroxylapatite has been reported to bring about an incorporation after the implantation which is initially more rapid than that obtained with, for example, pure titanium, see, for example, Gottlander et al. 1991. However, little is known about the long-term stability of bioactive implants. When a bioactive material is incorporated, it is thought that, while some of the bone formation starts on the traumatized bone surface, the bone formation also starts directly on the implant surface by means of the formation of a mineral layer. For a non-bioactive implant, bone formation also starts on the traumatized bone surface. It is assumed that the mineral layer on the bioactive implant surface is created by, in the main, calcium ions and phosphate ions being released from the implant surface and then, inter alia, being reprecipitated onto the surface from the biological fluid which has become supersaturated as a result of the ion release, a course of events which, in theory, can be influenced both by characteristics of the ion release and by characteristics of the surface onto which the mineral layer is precipitated. Different bioactive materials have also been found to give rise to mineral layers of different nature. It is also known that parameters, such as, for example, the crystallinity, of the bioactive material, for example hydroxylapatite, can influence the resulting structure of the interface between the implant and the host system. However, a change in crystallinity results in a change in both ion release and surface structure so that is difficult, in traditional experiments, to determine what has caused a particular biological response. Is it some specific surface property such as topography, leakage of calcium ions and/or phosphate ions, or synergistic effects between them, which gives the hydroxylapatite its implantation properties? Are the same effects obtained with another implant material, for example titanium, if a certain dose of calcium ions and/or phosphate ions is added during the implantation? How do added substances influence the anchorage of the implant over the long term?

The above examples demonstrate that the anchorage of an implant can be influenced by different implant-related factors. However, which factor or factors control (s) this influence is far from being elucidated. Consequently, in order to elucidate this, experiments are required in which individual factors can be varied independently of each other.

So-called diffusion chambers are also previously known, which chambers are, however, not used for studies bearing any similarity to the situation in the case of intraosseous clinical implants. The diffusion chamber consists of a plastic ring to whose two end surfaces have been cemented one or more semipermeable membranes, for example so-called Millipore membranes, of varying pore size. Different substances can be supplied to the chamber via a hole in the plastic ring, which hole is then sealed.

The diffusion chamber can, for example, be filled with cells of a certain type. After implanting the diffusion chamber in a host organism, these cells are then cultured in the biological medium which is created when certain of the substances which are present in the surrounding tissue diffuse in through the membrane, and the temperature, etc., follow the conditions in the surrounding tissue, see, for example, Johnson et al.1988, Dohi et al. 1993 and Kataoka and Urist 1993. In this case, therefore, the diffusion chamber is being used as an "incubator" in order to produce a certain environment, usually in soft tissue, thereby differing substantially from the invention both as regards application and design.

Transplants of certain cells in the diffusion chamber, or the supply of another biological substance by means of using a diffusion chamber, have also been found to have the ability to influence the tissue outside the chamber, see, for example, Nogami and Urist 1975. While experiments of that type can contribute to an increased understanding of general physiological processes, they do not have any obvious parallels with the incorporation of intraosseous clinical implants. In contrast to the previously known diffusion chamber, the invention resembles a clinical implant both as regards material properties, surface properties, surgical technique, location and use in the biological host system, i.e. the bone tissue in the present case. The previously known diffusion chamber differs from clinically employed intraosseous implants both as regards material properties, surface properties and surgical technique and also as regards location and use.

Zech et al.1993 implanted a teflon chamber having 3-mm diameter perforations subcutaneously in dogs, which were treated with oral doses of certain drugs after the implants had been incorporated. The contents of the chamber were compared with plasma concentrations as a function of time. Another exudate chamber was implanted by Eriksson et al. 1988 in order to investigate the inflammatory response around an implant. These chambers differ appreciably from the invention both as regards design and use. The exudate chambers are located in soft tissue, allow, as a result of their macroscopic perforations, free passage for even very large units such as cells, and are only used for sampling the biological fluid; not as a depot for added substances.

A similar model is described by Sunzel et al. 1990, who studied exudates in a steel-net chamber following the addition of zinc oxide in suspension, bacteria and/or polymorphonuclear cells. This chamber differs appreciably from the invention in its design and use.

### OBJECT AND CHARACTERISTICS OF THE INVENTION

The object of the invention is to produce a device which renders it possibly, directly at the interface between different implants and the tissue, to study and/or influence both the healing-in conditions and the conditions which pertain once primary incorporation has taken place, and to study, or make use of, how these conditions are influenced by different individual factors, especially those which are related to the surface itself (ion leakage, topography, microstructure, etc.), but also those which can consist of a cooperation between the properties of the surface and externally supplied components (ion, drugs, etc.). The device can, of course, be employed to study negative as well as positive effects, for example to determine whether chemical instability renders a certain material unsuitable for use as a biomaterial.

The device is characterized in that the surface of the anchoring element adjacent to the body tissue consist wholly or in part of a permeable material, a "filter", which allows added substances to diffuse out directly onto the implant surface, or to the biological fluid in the vicinity of the surface, from the central recess, with the anchoring element resembling clinically employed implants, or implants which are employed in research and development, with regard to material properties and/or surface properties, implantation site and surgical technique. "Filter" is consequently understood to mean, in this context, a material whose intentionally designed, open porosity allows certain substances to pass through it. Which these latter substances are is principally determined by the pore dimensions of the filter but also by chromatographic effects in the pores. By means of allowing different, added substances to diffuse out through the filter directly onto the surface of the implant or to the biological fluid around the implant in this way, the effect of ion release can be isolated from the other factors which are connected with the implant surface. In this way, known implant materials can be simulated (for example hydroxylapatite, where released calcium and phosphate may have a positive effect on the incorporation, or Ti6A14V, where, it is feared, aluminium may have a negative effect on the anchorage). It is also possible, in a development stage, to simulate novel implant materials which are specially designed to release a suitable bone-stimulating substance by means of allowing this substance to diffuse out from the depot via the filter. It is likewise possible to supply, via the filter, components which can have a positive effect on the healing-in, for example certain drugs or other chemically active substances. The filer furthermore allows substances from the surrounding tissue to diffuse, in the same way, into the central recess. This renders it possible to study the substance which diffuses into the central recess, in turn providing information on the conditions in the surrounding tissue during the incorporation and/or once the primary incorporation has taken place.

Externally, therefore, the device resembles a conventional intraosseous implant and can be applied using essentially the same surgical technique as used for conventional implants. By means of supplying certain substances from the depot to the tissue via the filter, the opportunity is provided, inter alia, of simulating implants which release certain substances rather then being chemically stable in the tissue. While this can be used, for example, in experimental studies to separate the ion release effect from other factors in the case of biomaterials which are already known (separation function), it can also be used to simulate novel biomaterials which are designed, for example, to release drugs or other healing-stimulating substances such as ions or biomolecules, etc. (addition function). The device also provides the opportunity of studying biochemical factors in the surrounding tissue by means of collecting the substance which diffuses into the depot from the biological host system (sampling function). In summary, the device offers completely novel possibilities for studying the interaction between an implant and its biological host system.

The effects of different substances on the long-term anchorage of the implant can be studied or utilized clinically by means of adding substances via the depot once incorporation has started.

If a certain substance can be demonstrated to have positive effects, the invention can then also be employed clinically. In this case, the macroscopic design of the implant has to be adapted in order to correspond to clinical demands for, for example, strength and compatibility with a suitable prosthetic system. The implant can either be designed with the inner depot being permanently sealed (closed depot) or so that the depot can be filled with substances during the implantation period (open depot). Such an implant can be provided with a surface which is stable over a long period, in contrast, for example, to conventional bioactive implants whose ion administration is caused by a slow resorption of the surface layer. With an open depot, the administration of different chemical substances over time can be controlled in an optimal manner.

### DESCRIPTION OF FUNCTION AND ILLUSTRATIVE EMBODIMENTS

In that which follows, the function of the invention, as well as some different embodiments of the invention, will be described in more detail while referring to the attached drawings which diagrammatically illustrate the invention and in which:
Figure 1 shows how the invention, placed in a biosystem, enables different functions, such as the separation function, the addition function and the sampling function, to be effected,
Figure 2 shows how the invention differs from the previously known harvesting chamber,
Figure 3 shows a first variant of the device in which the whole anchoring element consists of a filter,
Figure 4 shows another variant in which the bottom surface of the anchoring element consists of a filter, and
Figure 5 shows a third variant in which one or more porous inserts has/have been fitted into a conventional, screw-shaped implant.

### Description of function

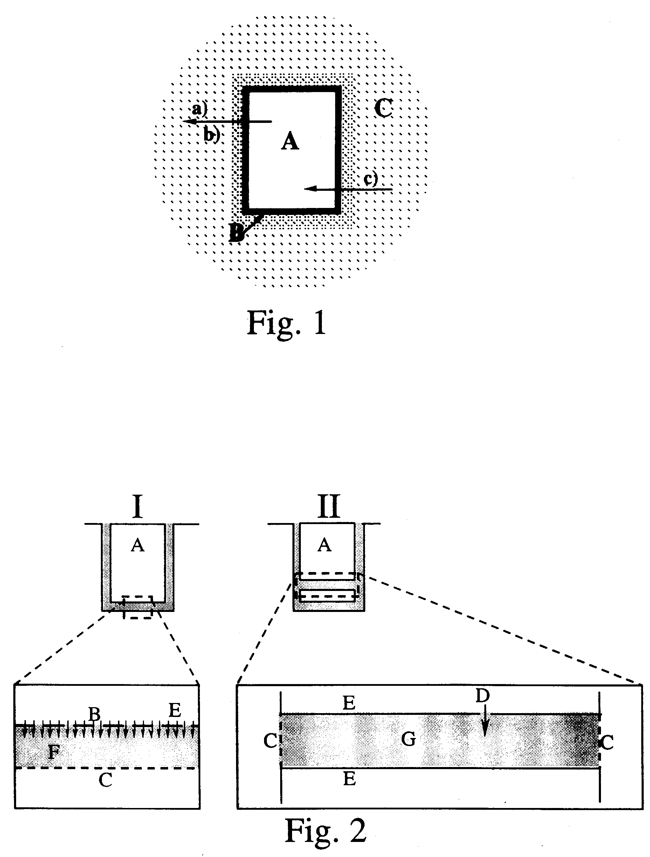

Figure 1 shows diagrammatically how the invention provides the opportunity of
a) being able to separate, in scientific studies of biomaterials which release ions, effects which are related to the surface of the material from effects which are related to released ions or other substances (separation function),
b) being able to supply, in scientific studies and/or clinical applications, components to the interface (ions, biomolecules, etc.) which positively influence the anchorage of the implant without introducing an unstable implant surface (addition function), and
c) being able to obtain samples, in the central recess, which reveal the composition of the external bioenvironment with regard to those components which can diffuse in through the filter (sampling function).

In Figure 1, A illustrates a depot, whose outer delimitation surface consists wholly or in part of a porous material B which is implanted in a biosystem C. Transport of substances out from the depot A to the biosystem C renders it possible to carry out experiments having a) a separation function and b) an addition function. Transport of substances from the biosystem C into the depot A renders it possible to carry out experiments having c) a sampling function. The addition function a) can be exploited clinically in order to influence the anchorage of the implant positively. The depot A can be closed or open.

In order to be able to exploit these possibilities fully, it is important that, even when it is being used in scientific experiments, the device as far as possible resembles clinical implants, or implants which are generally used in research. The device should, for example, be manufactured, wholly or in part, from materials which are relevant in clinical implant situations and, as far as possible, be given surface properties, for example topography and surface chemical composition, which are previously known in connection with implants. However, it can also, in certain situations, be relevant deliberately to design the device such that while it is made of previously used material it has novel surface properties, or vice versa. However, it should be pointed out that demands for permeability can sometimes result in a departure from the surface properties of a conventional implant as regards the permeable surface of the device, i.e. the filter surfaces. Previously known intraosseou implants often consists, wholly or in part, of metallic )Ti, Ti6A14V, steel etc.) or ceramic (Al₂O₃ etc.) material. Similarly, the geometrical shape of the device should be matched to conventional implants which are employed clinically or in research, as should the placing, both as regards site and surgical technique. In development situations, the device should, of course, be made to resemble the type of implant which is under development and hence not previously known.

The term "porous implant" is sometimes used in implant literature. It should be pointed out that this expression then normally relates to irregular, porous coatings of some material on a core which is otherwise impermeable. Normally, the purpose of these coatings is to increase the implant surface and to afford better mechanical locking. By contrast, the porous structure of the invention deliberately connects one space, the depot, with another, the biosystem, something which is not previously known from so-called "porous implants"

Unlike the previously known harvesting chamber II, which is described in SE 461 499, the filter surface B of the device I is in direct contact with the body tissue C after the installation, which is illustrated in Figure 2. The implant surface which the tissue "sees" then consists of the filter surface B and of the added substance, which diffuses out from the depot A. By contrast, in the known harvesting chamber II, the substance in question is supplied from the depot A via a capillary opening D, a so-called diffusion capillary, to the, initially empty, bone ingrowth channel G in order to study, for example, how rapidly new bone tissue can grow into this channel. In experiments in which the device I which is described here is implanted, the whole of the bone zone F under study abuts traumatized bone C, resembling the situation in clinical implantation. Likewise, the whole of the zone F under study abuts the surface B through which the substance in question is supplied. In experiments in which the previously known harvesting chamber II is implanted, only a small part of the bone surface G under study abuts traumatized bone C and the substance in question is supplied point-wise through the diffusion capillary D. Instead, the ingrowth zone G which is studied using the harvesting chamber II is surrounded by the implant surface E along the greater part of its boundary.

The previously known harvesting chamber is consequently employed in order to quantify and characterize an ingrowth zone G, i.e. the ingrowth of bone in a channel intended for this purpose, where appropriate under the influence of different stimuli, an arrangement which does not necessarily demonstrate how different implant materials incorporate into the tissue. By contrast, the invention I is used to study an incorporation zone F under the influence of different stimuli but otherwise under essentially the same conditions as when a clinical implant is incorporated.

### Description of illustrative embodiments

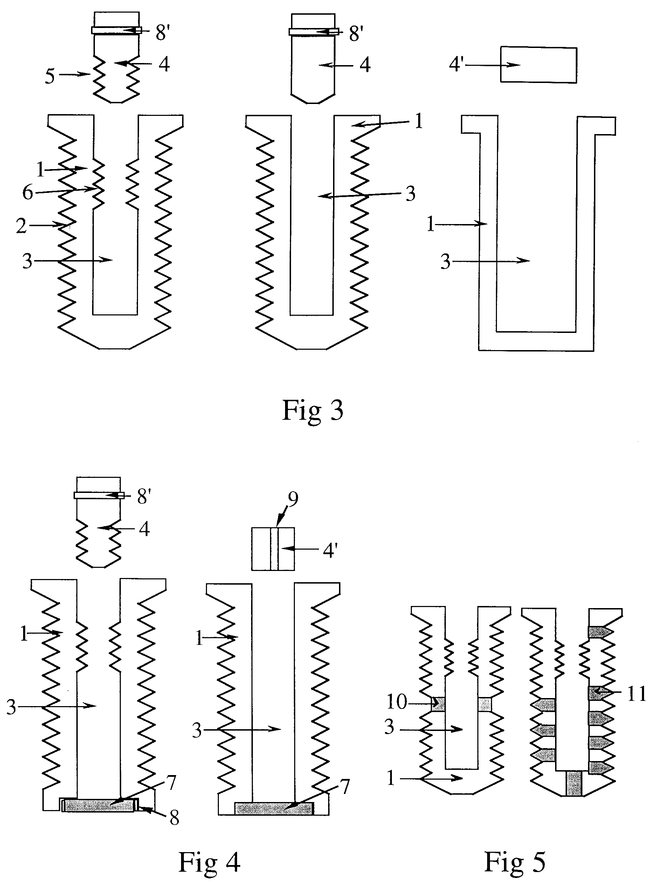

Figure 3 shows a first variant if the invention in which the actual filter is designed as an anchoring element 1, i.e. the geometric shape corresponds to a screw-shaped implant, a so-called fixture, having an external thread 2 and a central drilling-out cavity 3 which serves as the depot for the substance in question. Alternatively, the anchoring element 1 can be designed as a plug without a thread or else by only partially threaded. Both screw-shaped and plug-like intraosseous implants are currently employed clinically and in research.

In the variant which is shown in Figure 4, the anchoring element consists of a machined titanium screw 1 in which the lower surface of the screw has been replaced by a plane filter 7. The filter 7 thereby also closes off the central drilled-out cavity 3 for the substance which is added to the depot. The plane filter 7 can be sealed by means of a teflon seal 8 or, in certain cases, be welded or sintered into the anchoring element. While the actual anchoring element, the implant, is relatively simple to manufacture in this case, the experimental surface is limited to the lower surface of the implant. The filter 7 can be replaced by bottom plates which are coated with a variety of materials and/or with special topographical patterns which are difficult to produce on three-dimensional samples. These bottom plates do not need to be permeable insofar as the through transport of a particular substance is not part of the experiment. Otherwise, plates are difficult to evaluate biologically since the initial instability can create problems.

Figure 5 shows a third variant of the device in which the anchoring element consists of a conventional, machined screw implant where, however, an "insert" in the form of a plane, annular filter 10 in the form of a porous metal disc has been fitted. The insert can have the same structure as the porous plane filter 7. This implant can, for example, be anchored in the opposing cortex with the filter located in the medullary space in a long bone. In another variant, channels of porous material 11 are fitted into an otherwise conventional implant. These can, for example, be produced by drilling out cylindrical holes in the implant, filling these holes with a powder of suitable material and then sintering in this powder so that a structure having an open porosity is obtained. Alternatively, the channels 11 can be made and sintered in the raw material, for example a metal rod, after which the implants are turned or formed in another manner.

The filter can be made of a material which is normally used in implants (Ti, Al₂O₃, etc.) or of a material which is not normally used in biological situations ( Cu, Ni, SiC, etc.) It can, for example, be sintered, pressed or produced using another technique, for example an etching technique, so that an open porosity is obtained, which porosity is so fine-pored that, while it excludes certain units, for example cells, it allows the added substance to diffuse out through the filter material, and certain substances from the surrounding biosystem, for example from bone tissue, to diffuse in through the filter. Plane filters having a well-defined open porosity are obtainable commercially, for example for use in HPLC, high pressure liquid chromatography. Such filter (so-called "frits") can be used for implants in accordance with Figure 4 and 5. A variety of filtration membranes, for example so-called Millipore membranes, having a well-defined open porosity are also available commercially. Such membranes can also be used for the invention. If the whole or a part of the surface which faces the tissue following implantation has a different chemical composition from that which is desired, this surface can be coated with a thin layer of another material without the pores thereby being closed, for example by means of volatilization or sputter-coating.

The substance which is added to the depot can be of a type which has been shown to have, or which is assumed to have, a favourable effect on the anchorage (Ca²⁺, PO₄³⁻, growth hormones, etc.) or of a type which is suspected of being harmful. The implant surface which the tissue "sees" then consists of a material which, of itself, should not be biologically active, while the substance which has been supplied contributes to an environment which can be located close to a bioactive material. By systematically varying the filter material and the composition, concentration and total dose of the outwardly diffusing substance in biological experiments, a substantial understanding can be obtained of the mechanism involved in the incorporation or permanent anchorage of an implant.

The active substance can be added in solid or liquid form to the depot 3 and can, if required, be replenished during the course of the experiment. In this case, the sealing cap 4, 4' which normally closes off the depot has to be removed (open depot). The sealing cap 4 can, for example, be made of titanium and have an external thread 5 which engages in an internally threaded section 6 in the recess 3 in the anchoring element, and also an O-ring 8' as the sealing element. The recess 3 can also be entirely smooth-bored, in which case a sealing plug 4 is applied by means of forced fit. The sealing plug 4' can also be made of a material which is of itself sealing, for example teflon, in which cases no O-ring 8' is applied. A channel 9 can be drilled in this sealing plug so that a liquid substance can be supplied via a cannula which is passed into the channel of the sealing plug. If the plug is made of a sealing material and the diameter of the channel is somewhat less then the cannula which is used, a positive pressure can be applied in the depot when injecting the liquid substance so that the pores of the device are at least partially filled with the liquid substance.

The device can also be designed with a closed depot, i.e. so that the sealing cap cannot be removed. In those cases in which the device consists of both impermeable and permeable parts (Figs. 4 and 5), the substance in question can be introduced into the depot before it is sealed by the "filter" or "filters".

In experimental studies, the incorporation of the anchoring element in the tissue can be evaluated in a conventional manner, i.e. with the screw together with surrounding bone tissue being removed and sectioned after a variable healing period. Comparison is made with a conventional implant which does not have a filter and which does not have a surface coating of the diffusing-through substance, with a filter implant which has been filled with, for example, physiological saline solution (placebo) or has been surface-coated with the added substance, or with an implant which has a filter which has been manufactured from a completely different material.

The invention is not limited to the three variants which have been described here and can be varied within the scope of the subsequent patent claims. When the invention is used in clinical applications, the implant should be adapted in order to conform to relevant strength requirements and compatibility with a suitable prosthetic system.

### REFERENCES

Dohi Y, Sugimoto K, Yoshikawa T, Ohgushi H, Katsuda T, Tabata and Moriyama T: "Effect of cadmium on osteogenesis within diffusion chambers by bone marrow cells: Biochemical evidence of decreased bone formation capacity", Toxicol Appl Pharmacol 120: 274-280 (1993).

Eriksson A, Bjursten L M, Eriksson L E and Thomsen P: "Hollow implants in soft tissues allowing quantitative studies of cells and fluid at the implant interface, Biomaterials 9:, 86-90 (1988).

Gottlander M and Albrektsson T: "Histomorphometric studies of hydroxylapatite-coated and uncoated cp titanium threaded implants on bone", Int J Oral Maxillofac Impl 6: 399-404 (1991).

Johnson K A, Howlett C R, Bellenger C R and Armati-Gulson P: "Osteogenesis by canine and rabbit bone marrow in diffusion chambers", Calcif Tiss Int 42: 113-118 (1988).

Kataoka H and Urist M R: "Transplant of bone marrow and muscle-derived connective tissue cell cultures in diffusion chambers for bioassay of bone morphogenetic protein", Clin Orthop & Rel Res; 286: 262-70 (1993).

Nogami H and Urist M R: "Transmembrane bone matrix gelatin-induced differentiation of bone", Calcif Tissue Res 19: 153-163, (1975).

Sunzel B, Lasek J, Söderberg T, Elmros T, Hallmans G and Holms S: "The effect of zinc oxide on staphylococcus aureus and polymorphonuclear cells in a tissue cage model", Scand J Plast Reconstr Hand Surg 24: 31-35 (1990).

## Claims

1. Device for studying and/or influencing the anchorage of implants in living biological tissue under various conditions, especially as a response to locally supplied substances, with the device consisting of an anchoring element for fastening in the tissue, preferably bone tissue, and a central recess in the form of a depot for supplying a substance whose effect on the anchorage of the implant in the tissue is to be studied or utilized, characterized in that the surface of the anchoring element (1) adjacent to the tissue consists wholly or in part of a permeable material, "filter", which allows added substances to diffuse out directly onto the implant surface, or into the biological fluid around the implant surface, from the central recess (3), with the anchoring element resembling clinically employed implants, or implants which are employed within research and development, as regards material properties and/or surface properties, implantation site and surgical technique.

2. Device according to Patent Claim 1, characterized in that the permeable material also allows substances from the surrounding tissue to diffuse, in the same way, into the central recess (3).

3. Device according to Patent Claim 1, characterized in that the anchoring element (1) is made wholly or in part of a permeable metallic or ceramic material.

4. Device according to Patent Claim 3, characterized in that the permeable material has an open porosity which affords a deliberate connection between the central recess (3) and the surface of the anchoring element (1) adjacent to the tissue in order to permit the diffusion of certain substances outwards or inwards through the material.

5. Device according to Patent Claim 4, characterized in that the whole of the anchoring element (1) is made of a filter material in which the open porosity has been produced by means of sintering, pressing, etching or a similar technique.

6. Device according to Patent Claim 1, characterized in that the bottom surface of the anchoring element (1) consists of a filter (7), preferably a plane filter having a well-defined open porosity.

7. Device according to Patent Claim 1, characterized in that the anchoring element (1) includes one or more annular filters (10) having a well-defined open porosity which have been fitted as inserts into an otherwise conventional implant.

8. Device according to Patent Claim 1, characterized in that the anchoring element (1) includes one or more radial channels which are filled with a porous material (11), filter, having a well-defined open porosity, which channels are fitted into an otherwise conventional implant.

9. Device according to Patent Claim 1, characterized in that the depot (3) is normally closed by a sealing cap (4) which can be removed when replenishing the active substance.
